# EUROPEAN PATENT APPLICATION

(11) **EP 2 706 067 A1**
(43) Date of publication of application: **12.03.2014**
(21) Application number: 12183268.7
(22) Date of filing: 06.09.2012
(51) Int. Cl.: C07K 14/435, A61K 35/74, A61K 38/57

(54) **Probiotic bacteria as carrier for a helminth-derived immunomodulator for the treatment of inflammatory disorders**

(71) Applicant: Humboldt-Universität zu Berlin, 10099 Berlin (DE); Freie Universität Berlin, 14195 Berlin (DE)
(72) Inventor: Hartmann, Susanne, 12307 Berlin (DE); Rausch, Sebastian, 10997 Berlin (DE); Whelan, Rose, 10827 Berlin (DE); Wieler, Lothar H., 14109 Berlin (DE)
(74) Representative: Gulde Hengelhaupt Ziebig & Schneider

(57) **Abstract**

The present invention is directed to a transgenic probiotic microorganism genetically engineered to express and secrete cystatin, wherein said cystatin is preferably comprises or consists of:
- an amino acid sequence with SEQ ID NO. 1 to 3; and
- an amino acid sequence that is at least 70% identical, preferably at least 80% identical, more preferably at least 90% identical and most preferably at least 99% identical to an amino acid sequence with SEQ ID NO. 1 to 3, and to its use for treatment of gastrointestinal inflammatory diseases.

SEQUENCE LISTING

<110>  Humboldt-Universität zu Berlin
       Freie Universität Berlin

<120>  Probiotic bacteria as carrier for a helminth-derived
       immunomodulator for the treatment of inflammatory disorders

<130>  P09932EP

<160>  10

<170>  PatentIn version 3.5

<210>  1
<211>  157
<212>  PRT
<213>  Acanthocheilonema viteae

<400>  1

Met Met Leu Ser Ile Lys Glu Asp Gly Leu Leu Val Val Leu Leu Leu
1               5                   10                  15

Ser Phe Gly Val Thr Thr Val Leu Val Arg Cys Glu Glu Pro Ala Asn
            20                  25                  30

Met Glu Ser Glu Val Gln Ala Pro Asn Leu Leu Gly Gly Trp Gln Glu
        35                  40                  45

Arg Asn Pro Glu Glu Lys Glu Ile Gln Asp Leu Leu Pro Lys Val Leu
    50                  55                  60

Ile Lys Leu Asn Gln Leu Ser Asn Val Glu Tyr His Leu Met Pro Ile
65                  70                  75                  80

Lys Leu Leu Lys Val Ser Ser Gln Val Val Ala Gly Leu Arg Tyr Lys
                85                  90                  95

Met Glu Ile Gln Val Ala Gln Ser Glu Cys Lys Lys Ser Ser Gly Glu
            100                 105                 110

Glu Val Asn Leu Lys Thr Cys Lys Arg Leu Glu Gly His Pro Asp Gln
        115                 120                 125

Ile Ile Thr Leu Glu Ala Trp Glu Lys Ser Trp Glu Asn Phe Leu Gln
    130                 135                 140

Val Lys Ile Leu Glu Lys Lys Glu Val Leu Ser Ser Val
145                 150                 155

<210>  2
<211>  162
<212>  PRT
<213>  Onchocerca volvulus

<400>  2

Met Leu Thr Ile Lys Asp Gly Thr Leu Leu Ile His Leu Leu Leu Phe
1               5                   10                  15

Ser Val Val Ala Leu Val Gln Leu Gln Gly Ala Lys Ser Ala Arg Ala
            20                  25                  30

Lys Asn Pro Ser Lys Met Glu Ser Lys Thr Gly Glu Asn Gln Asp Arg
        35                  40                  45

Pro Val Leu Leu Gly Gly Trp Glu Asp Arg Asp Pro Lys Asp Glu Glu
    50                  55                  60

Ile Leu Glu Leu Leu Pro Ser Ile Leu Met Lys Val Asn Glu Gln Ser
65                  70                  75                  80

Asn Asp Glu Tyr His Leu Met Pro Ile Lys Leu Leu Lys Val Ser Ser
                85                  90                  95

Gln Val Val Ala Gly Val Lys Tyr Lys Met Asp Val Gln Val Ala Arg
            100                 105                 110

Ser Gln Cys Lys Lys Ser Ser Asn Glu Lys Val Asp Leu Thr Lys Cys
        115                 120                 125

Lys Lys Leu Glu Gly His Pro Glu Lys Val Met Thr Leu Glu Val Trp
    130                 135                 140

Glu Lys Pro Trp Glu Asn Phe Met Arg Val Glu Ile Leu Gly Thr Lys
145                 150                 155                 160

Glu Val



<210>  3
<211>  161
<212>  PRT
<213>  Brugia malayi

<400>  3

Met Met Ser Thr Met Ser Ile Lys Glu Gly Leu Leu Val Ile Leu Leu
1               5                   10                  15

Ser Leu Phe Leu Phe Asp Thr Thr Ala Leu Ile His Arg Arg Glu Ile
            20                  25                  30

Pro His Met Glu Ser Lys Gly Gln Met Gln Arg Gly Gln Val Leu Leu
        35                  40                  45

Gly Gly Trp Gln Glu Arg Ser Pro Glu Asp Asn Glu Ile Leu Glu Leu
    50                  55                  60

Leu Pro Ser Val Leu Thr Lys Val Asn Gln Gln Ser Asn Asp Glu Tyr
65                  70                  75                  80

His Leu Met Pro Ile Lys Leu Leu Lys Val Ser Ser Gln Val Val Ala
                85                  90                  95

Gly Val Lys Tyr Lys Met Glu Val Gln Val Ala Arg Ser Glu Cys Lys
            100                 105                 110

Lys Ser Ala Ser Glu Gln Val Asn Leu Lys Thr Cys Lys Lys Leu Glu
        115                 120                 125

Gly His Pro Asp Gln Val Met Thr Leu Glu Val Trp Glu Lys Pro Trp
    130                 135                 140

Glu Asp Phe Leu Gln Val Asn Ile Leu Glu Thr Lys Val Leu Ser Ser
145                 150                 155                 160

Val

<210>  4
<211>  687
<212>  DNA
<213>  Acanthocheilonema viteae

<400>  4
aataaacaaa gctaacgacg atatgatgtt gtcaataaag gaggatggat tgttggtggt      60

acttttattg tcgttcggtg tgacgacagt tttggtgcgc tgtgaagaac ccgcaaatat     120

ggaatctgag gtacaagcgc ccaatttatt gggaggatgg caggaacgca atccggaaga     180

gaaagaaata caggacctgt tgccaaaggt attaattaaa ctaaatcagc tgtcaaacgt     240

ggagtaccac ctaatgccaa tcaaattact gaaagtttca tctcaagttg tggctggttt     300

gagatacaag atggaaatac aggttgctca atcagaatgc aaaaaaagtt caggcgagga     360

agttaatctg aaaacatgta aaagattgga aggacatccg gatcagatta tcacgttgga     420

ggcatgggag aaatcatggg aaaatttttt gcaagtcaaa attctggaaa aaaaagaagt     480

actctcatca gtgtgattct ttttgcaagt ctcacttaac gttatccact gtgctttgta     540

gtatttttat tgtttaatgc ttacgatttt ttttaaaatg cattttgtaa ttttatcaaa     600

atcaaaatgg tattgtaatt ggtgcaaaaa atatttaaag ttgataatgc caatattcca     660

gcttataatt gttaaagtac attgtga                                         687

<210>  5

<211>  26
<212>  DNA
<213>  artificial

<220>
<223>  primer AvCystatin

<400>  5
acgtatgcat tggtgcgctg tgaaga                                            26


<210>  6
<211>  27
<212>  DNA
<213>  artificial

<220>
<223>  primer AvCystatin

<400>  6
acgtatgcat tcactgatga gagtact                                           27


<210>  7
<211>  33
<212>  DNA
<213>  artificial

<220>
<223>  primer AvCystatin

<400>  7
tcgtgtcgac ggttttggtg cgctgtgaag aac                                    33


<210>  8
<211>  29
<212>  DNA
<213>  artificial

<220>
<223>  primer AvCystatin

<400>  8
acatgcggcc gctcacactg atgagagta                                         29


<210>  9
<211>  33
<212>  DNA
<213>  artificial

<220>
<223>  primer AvCystatin

<400>  9
tcgtgtcgac ggttttggtg cgctgtgaag aac                                    33


<210>  10
<211>  29
<212>  DNA
<213>  artificial

<220>

<223>  primer AvCystatin

<400>  10
acatgcggcc gctcacactg atgagagta                                          29

## Description

About 3.6 million Europeans and US-Americans suffer from Crohn's disease (CD) or ulcerative colitis (UC), collectively known as inflammatory bowel diseases (IBD). Both CD and UC are chronic inflammatory disorders characterized by periods of remission and relapse with subsequently unpredictable fluctuations in symptom severity. Symptoms can occur at any age, but the onset generally appears between the ages of 15 and 35 and can then severely affect the quality of life for sufferers. Symptoms include permanent diarrhoea, cramping, abdominal pain, weight loss, fatigue and rectal bleeding during disease flare-ups. Common complications, especially in CD patients, are formation of deep ulcers with high risk of intestinal ruptures, or intestinal blockade due to swelling and scar tissue. Areas other than the intestine such as the joints, skin, eyes and liver can also be affected (http://www.cdc.gov/ibd/).

In IBD, the immune system mistakes bacterial components, food or other materials in the intestine for foreign substances and finally attacks the cells of the intestine. Many potential factors are involved in the induction and propagation of IBD, such as inheritance of gene loci linked to IBD susceptibility and life style associated factors, such as diet and smoking. The intestinal bacterial flora is central for the initiation of IBD development, but eventually activation of the adaptive immune system leads to the strong production of inflammatory cytokines by T helper cells, which constitute the dominant force driving chronic inflammation.

A wide range of therapies are used to treat IBD symptoms as the numerous factors involved in the initiation and propagation of IBD mean that response to specific treatments varies greatly between individual patients. Antibiotic treatment may temporarily improve the symptoms by reducing the gastro-intestinal bacterial load, but may also lead to malnourishment or exacerbate the condition by allowing resistant pathogens to flourish in the less competitive environment. Patients are routinely treated with corticosteroids, which help reduce inflammation. Corticosteroid treatment can be highly effective when administered long term, however it also results in the highest adverse effect incidences. Up to 50% of patients treated with corticosteroids develop side effects ranging from metabolic disorders such as glucose intolerance, malnourishment, dermatological and mood/sleep disturbances, to osteoporosis, and finally resistance to treatment with long term usage. Corticosteroid sparing therapies, such as treatment with immunosuppressants are routinely administered in order to decrease the risk of side effects; however efficacy may also be decreased. The use of a steroid-sparing agent can induce remission in CD patients and when administered for prolonged periods can even provide long-term maintenance of remission. However, monoclonal antibody treatments (e. g. Infliximab targeting the inflammatory cytokine TNF-alpha) are very cost-intensive, especially when the need for long-term treatment is considered and TNF -alpha inhibition therapy can result in serious side effects.

The application of probiotic microorganisms has been proposed to have beneficial effects alone or in conjunction with conventional treatments in IBD patients. A summary of current probiotic based treatment approaches is provided by Mack et al. (Nutrients 2011, 3, 245-24). While application of *Lactococcus lactis* alone was insufficient for treatment of human IBD, transgenically modified *L. lactis* strains expressing immunosuppressants such as interleukin-(IL-) 10 and anti-TNF-alpha ameliorated colitis in mouse models. However, a recent metaanalysis of human IL-10 treatment has determined no significant effect in IBD patients compared to the placebo control.

Clearly the current treatments are less than ideal as they are often accompanied by severe side effects and while they may help to control symptoms and maintain remission, they offer no cure to the disease. Additionally many patients are refractory or, over time, respond insufficiently to the available medications. Thus, two thirds to three quarters of patients will at some point in their lives require medical surgery to remove heavily inflamed parts of the intestine or even the entire colon (http://www.cdc.gov/ibd/). Both forms of IBD thus generally affect patients for life and new treatments are urgently needed.

According to the present invention, a transgenic probiotic microorganism is provided which is genetically engineered to express and secrete nematode derived cystatin.

Recently, an effective immune modulator has been identified, a cystatin derived from parasitic nematode. Nematode derived cystatin efficiently suppresses host immune responses in order to maintain survival of the nematode in the host. Nematode derived cystatin down-regulates inflammation and therefore ameliorates inflammatory pathologies such as airway hyper-reactivity and gastro-intestinal inflammatory diseases like e.g. colitis *in vivo* in murine models, see WO 2008/015014 A2. Macrophages are the target cell of nematode derived cystatin, acquiring a regulatory phenotype when exposed to the recombinant protein (Klotz et al., "A helminth immunomodulator exploits host singaling events to regulate cytokine production in macrophages", PLoS pathogens 2011, 7(1)). Macrophages exposed to nematode cystatin were also found to suppress inflammation when transferred to mice with airway hyper reactivity or gastro-intestinal inflammatory diseases like e.g. colitis, independent of the recipient mice ever being exposed to the protein.

A new application method for nematode derived cystatin has been developed as it enables for constant, prolonged exposure of such cystatin to the inflamed gut. Previously, nematode derived cystatin was given in relatively large doses as an intra-peritoneal injection, which significantly suppressed inflammation in a murine colitis model (WO 2008/015014 A2). This application method is unrealistic in human gastro-intestinal inflammatory diseases like e.g. IBD. The creation of transgenic probiotic microorganisms capable of expressing a highly efficient immunomodulator of parasitic origin is a novel approach aiming to improve therapy of IBD and other gastro-intestinal inflammatory disorders. Administration of the transgenic probiotic microorganism allows for long term secretion of the cystatin immune modulator in the gut at the site of inflammation, without exposing the recipient to a parasitic infection. The transgenic probiotic microorganism also bypasses the need to apply large amounts of protein orally, in which maintenance of protein integrity through the digestive process is a concern or other more systemic routes. Therefore, this innovative therapy combines the inherent beneficial effects of probiotic microorganisms with the additional potential of the nematode derived cystatin in a safe and localized manner.

As a new, innovative approach for long term application of the highly efficient nematode derived cystatin and treatment of gastro-intestinal inflammatory diseases like e.g. IBD, a transgenic probiotic microorganism is provided which is capable of expressing and secreting the nematode derived immune modulator. This transgenic probiotic excellently combines the already known effects of probiotic microorganisms like e.g. of *Echerichia coli* Nissle (EcN) and nematode derived cystatin in the treatment of gastro-intestinal inflammatory diseases like e.g. colitis in a surprisingly beneficial way, while allowing for a cost effective and efficient therapy directly at the site of inflammation. The benefits are not impeded by any adverse effects, as no significant changes to the host immune parameters or health status were observed when healthy young piglets (as a model of the human gastrointestinal and immune system) were treated continually with the transgenic probiotic over a two week period. This transgenic probiotic therefore provides an ideal therapy for gastro-intestinal inflammatory diseases like e.g. IBD in a market clearly lacking a safe, effective, cost efficient treatment for a morbid disease with rising incidence in the industrialized and developing world.

The present invention is directed to a probiotic microorganism.

The term "microorganism" is used herein in its art recognised meaning and refers in particular to organisms selected from bacteria, archaea, unicellular fungi like e.g. yeast, algae and unicellular protozoa. Preferably, the term "microorganism" is used to denote bacteria and unicellular yeast.

For the purpose of the present invention, the term "probiotic" refers to a microorganism capable of exerting a beneficial effect upon administration to a suitable subject or host. Upon proper administration, a probiotic microorganism is capable to survive in said subject or host for a considerable period of time. In particular, the probiotic microorganism of the invention refers to a vital microorganism which, upon proper administration to said subject or host, is capable of proliferation and/or colonization within said subject or host. The skilled person is well aware of microorganisms that can be used as probiotics in the sense of the present invention. There is also ample literature available describing probiotic microorganisms used for treatment of numerous diseases like e.g. gastro-intestinal inflammatory diseases. A recent overview on the state of the art in this field is given by Mack et al (2011). Preferably the probiotic microorganism of the invention is a microorganism selected from the bacterial genus lactobacillus, bifidobacterium, escherichia and/or from the yeast genus saccharomyces. It appears to be particularly beneficial if the probiotic microorganism is selected from probiotic microorganisms which have already been used for treatment of a disease like e.g. a gastro-intestinal inflammatory disease. Thus, the probiotic microorganism of the invention may be selected from *Bifidobacterium longum, Bifidobacterium breve, Bifidobacterium bifidum, Bifidobacterium infantis, Bifidobacterium animalis (Bifidobacterium lactis), Bifidobacterium animalis (Bifidobacterium lactis) strain Bb12, Lactobacillus acidophilus, Lactobacillus acidophilus strain La5, Lactobacillus casei, Lactobacillus johnsonii, Lactobacillus johnsonii strain LA1, Lactobacillus raffinolactis, Lactobacillus lactis, Lactobacillus salivarius, Lactobacillus plantarum, Lactobacillus paracasei, Lactobacillus bulgaricus, Lactobacillus rhamnosus, Lactobacillus rhamnosus strain GG, , Escherichia coli, Escherichia coli strain Nissle, Escherichia coli strain Nissle 1917 (EcN), Enterococcus durans, Faecalibacterium prausnitzii, Pediococcus pentoseceus, Saccharomyces thermophilus and*/*or Saccharomyces boulardii.* In a particularly preferred embodiment, the probiotic microorganism of the invention is *Escherichia coli* strain Nissle 1917 (EcN). *Escherichia coli* Nissle 1917 (EcN) was first applied in 1917 as a therapy for gastro-intestinal disease in soldiers and has been available for use in humans since (Nissle et al., Zeitschrift für klinische Medizin 1951, 2 (3-4); 68). Several recent trials have observed significant responses to treatment with the probiotic EcN in gastrointestinal disease in infants and children as well as when rectally administered during acute flare-ups of ulcerative colitis. The colonization properties in humans and lack of immune activation by the bacterium make EcN an excellent carrier for immunosuppressive factors for treatment of gastro-intestinal inflammatory diseases like e.g. IBD. Thus, *Eschericia coli* Nissle 1917 has proven significant beneficial effects as therapeutic for ulcerative colitis. EcN effectively colonizes the gut without inducing immune activation in the host and is, therefore, an excellent carrier for the nematode derived immune modulator cystatin.

According to the present invention the probiotic microorganism is transgenic in that it is genetically engineered to express and secrete nematode derived cystatin. Cystatins are proteins of a family of cysteine protease inhibitors which contain four conserved cysteine residues supposed to be involved in disulfide bond formation. Nematode derived cystatins can be cystatins that are identical in sequence to cystatins derived from or originally described for nematodes. Nematode derived cystatins exhibit significant binding to the surface marker CD36. A suitable assay for testing CD36 binding capabilities is described in WO 2008/015014 A2. Nematode derived cystatins show binding to CHO cells transfected to express CD36, whereas nematode derived cystatins show no significant binding to CHO cells lacking CD36 expression.

Nematode derived cystatin ameliorates inflammation in murine models of colitis when injected intra-peritoneally, see e.g. WO 2008/015014 A2. The effect is mediated by the induction of regulatory cell types and an anti-inflammatory cytokine milieu.

The nematode derived cystatin expressed and secreted by the transgenic probiotic microorganism of the invention may comprise or consist of:
- an amino acid sequence with SEQ ID NO. 1, 2 or 3; and/or
- an amino acid sequence that is at least 70% identical, preferably at least 80% identical, more preferably at least 90% identical and most preferably at least 99% identical to an amino acid sequence with SEQ ID NO. 1, 2 or 3.

SEQ ID NO. 1 denotes the amino acid sequence of Acanthocheilonema *viteae* cystatin, also called AvCystatin, with the Gene bank accession No. L43053.

SEQ ID NO. 2 denotes the amino acid sequence of *Onchocerca volvulus* cystatin with the Gene bank accession No. M37105.

SEQ ID NO. 3 denotes the amino acid sequence of *Brugia malayi* cystatin with Gene bank accession No. AF 177193_1.

The nematode derived cystatin may also comprise or consist of an amino acid sequence that is at least 70% identical, preferably at least 80% identical, more preferably at least 90% identical and most preferably at least 99% identical to an amino acid sequence with SEQ ID NO. 1, 2 or 3. Preferably, such nematode derived cystatins contain the four conserved cysteine residues of the cystatin family and bind to CD36.

Preferably, the nematode derived cystatin expressed and secreted by the transgenic probiotic microorganism of the invention comprises or consists of:
- an amino acid sequence with SEQ ID NO. 1; and/or
- an amino acid sequence that is at least 70% identical, preferably at least 80% identical, more preferably at least 90% identical and most preferably at least 99% identical to an amino acid sequence with SEQ ID NO. 1. Preferably, nematode derived cystatins comprising or consisting of an amino acid sequence that is at least 70% identical, preferably at least 80% identical, more preferably at least 90% identical, and most preferably at least 99% identical to an amino acid sequence with SEQ ID NO. 1 contain the four conserved cysteine residues of the cystatin family and bind to CD36.

The transgenic probiotic microorganism of the invention is genetically engineered to express and secrete nematode derived cystatin. The term "transgenic probiotic microorganism" in the sense of the present invention refers to probiotic microorganism which itself have been genetically modified to express and secrete nematode derived cystatin or to progeny thereof which still expresses and secretes nematode derived cystatin.

Starting from the amino acid sequence of the nematode derived cystatin, the skilled person is well aware of how to produce transgenic probiotic microorganisms of the invention. In general terms such transgenic probiotic microorganisms may be produced by cloning a nucleic acid molecule encoding for the desired nematode derived cystatin amino acid sequence into a vector molecule which allows for expression and secretion of the expression product and to transform or transfect a probiotic microorganism with said vector molecule. The skilled person is well aware of the degeneracy of the genetic code and knows that there is more than one possibility to design a nucleic acid molecule encoding for a given amino acid sequence. The amino acid sequence of *Acanthocheilonema viteae* cystatin with SEQ ID NO. 1, also called AvCystatin, may be expressed using a nucleic acid molecule comprising or consisting of the nucleic acid sequence with SEQ ID NO. 4, see nucleic acid sequence of *Acanthocheilonema viteae* cystatin, also called AvCystatin, with the Gene bank accession No. L43053.

The transgenic probiotic microorganism may be stably or transiently transformed or transfected to express and secrete the desired nematode derived cystatin, preferably the transgenic probiotic microorganism is genetically engineered to stable express and secrete the desired nematode derived cystatin.

The present invention is also directed to a pharmaceutical composition comprising a transgenic probiotic microorganism of the invention and at least one pharmaceutically acceptable excipient.

The transgenic probiotic microorganism or the pharmaceutical composition of the invention may be provided in encapsulated form, may be used as food supplement or may be incorporated into food products.

The transgenic probiotic microorganism of the invention and the pharmaceutical composition of the invention can be used in treatment of gastro-intestinal inflammatory diseases.

As used herein, the term "treating" refers to reversing, alleviating or inhibiting the progress of a disease, disorder or condition, or one or more symptoms of such disease, disorder or condition, to which such term applies. As used herein, "treating" may also refer to decreasing the probability or incidence of the occurrence of a disease, disorder or condition in a mammal as compared to an untreated control population, or as compared to the same mammal prior to treatment. For example, as used herein, "treating" may refer to preventing a disease, disorder or condition, and may include delaying or preventing the onset of a disease, disorder or condition, or delaying or preventing the symptoms associated with a disease, disorder or condition. As used herein, "treating" may also refer to reducing the severity of a disease, disorder or condition or symptoms associated with such disease, disorder or condition prior to a mammal's affliction with the disease, disorder or condition. Such prevention or reduction of the severity of a disease, disorder or condition prior to affliction relates to the administration of the composition of the present invention, as described herein, to a subject that is not at the time of administration afflicted with the disease, disorder or condition. As used herein "treating" may also refer to preventing the recurrence of a disease, disorder or condition or of one or more symptoms associated with such disease, disorder or condition. The terms "treatment" and "therapeutically," as used herein, refer to the act of treating, as "treating" is defined above.

The transgenic probiotic microorganism and the pharmaceutical composition of the invention are intended for use in treatment of gastro-intestinal inflammatory diseases. Gastro-intestinal inflammatory diseases comprise diseases of the gastro-intestinal tract wherein an inflammatory component is involved like e.g. inflammatory bowel diseases (IBD). The main forms of IBD are Crohn's disease (CD) and ulcerative colitis (UC). Other forms of IBD comprise Collagenous colitis, Lymphocytic colitis, Ischaemic colitis, Diversion colitis, Behçet's disease and Indeterminate colitis.

Preferably, the transgenic probiotic microorganism of the invention and the pharmaceutical composition of the invention are used in treatment of inflammatory bowel diseases. Particularly, the transgenic probiotic microorganism of the invention and the pharmaceutical composition of the invention are used in treatment of Crohn's disease and/or ulcerative colitis.

According to another aspect, the present invention is directed to the use of a transgenic probiotic microorganism of the invention or a pharmaceutical composition of the invention in the manufacture of a medicament for treatment of gastro-intestinal inflammatory diseases, preferably of inflammatory bowel disease, even more preferably of Crohn's disease and/or ulcerative colitis.

The present invention is also directed to a method of treatment of gastro-inflammatory diseases, wherein a subject in need of such treatment is administered an effective amount of a transgenic probiotic microorganism of the invention or of a pharmaceutical composition of the invention.

According to the present invention, the transgenic probiotic microorganism or the pharmaceutical composition of the invention is administered preferably at an effective amount. An "effective amount" is the dose of the transgenic probiotic microorganism or the pharmaceutical composition that upon administration to a patient yields a measurable therapeutical effect with regard to the disease of interest. In the present invention an effective amount is the dose of the transgenic probiotic microorganism or the pharmaceutical composition that upon administration to a patient yields a therapeutic effect with regard to a gastro-inflammatory disease, like e.g. IBD.

The gastro-inflammatory diseases to be treated by the method of the invention are preferably inflammatory bowel disease, even more preferably Crohn's disease and/or ulcerative colitis.

As the current standard treatments for IBD are often ineffective, we have developed a strategy combining the highly effective anti-inflammatory potential of a parasite immunomodulator (AvCystatin) with the beneficial effects of a probiotic bacterium *(Escherichia coli* Nissle 1917). The bacterium is genetically engineered to produce the parasite protein and upon intestinal colonization allows for the direct and prolonged delivery of this potent immune modulator to the inflamed intestine. This excellent, cost-effective new strategy of treating IBD has the potential to support or replace standard treatments.

### FIGURES:

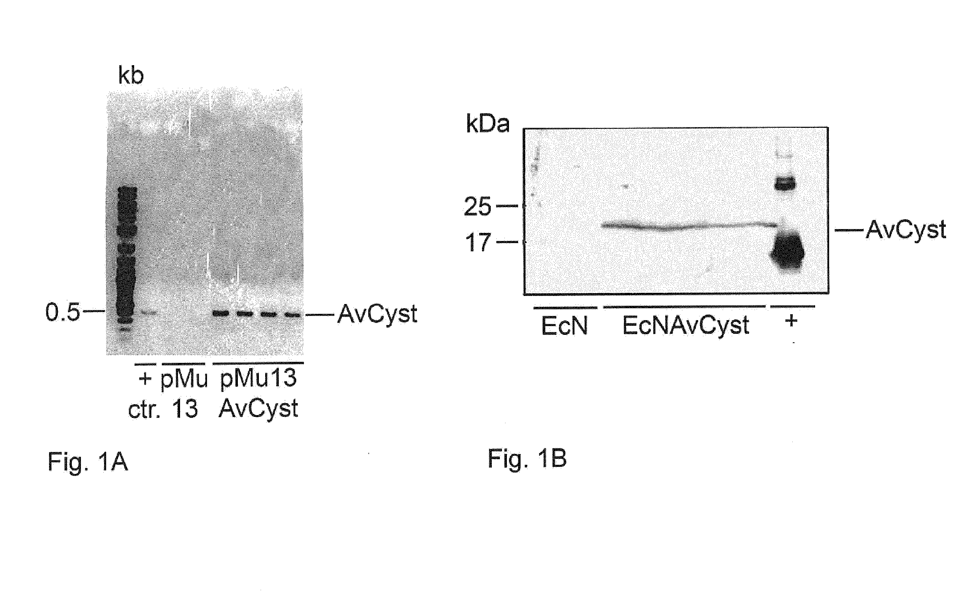

- **Figure 1:**: shows evidence of successful ligation and transformation of *A. viteae* Cystatin encoding gene into an *E. coli* Nissle 1917 carrier. Polymerase Chain Reaction with *A. viteae* Cystatin (AvCyst) specific primers successfully amplified a region the AvCyst gene ligated in the modified plasmid (pMU13AvCyst) but not in the plasmid lacking the gene insert (pMu13)(**A**). Western blot analysis with AvCyst specific antibodies of cell culture supernatant shows AvCyst in supernatant of the transgenically modified *Escherichia coli* Nissle 1917 (EcNAvCyst) but not from supernatant of control *E. coli* Nissle 1917 transformed with empty pMu13 plasmid (EcN) **(B).**

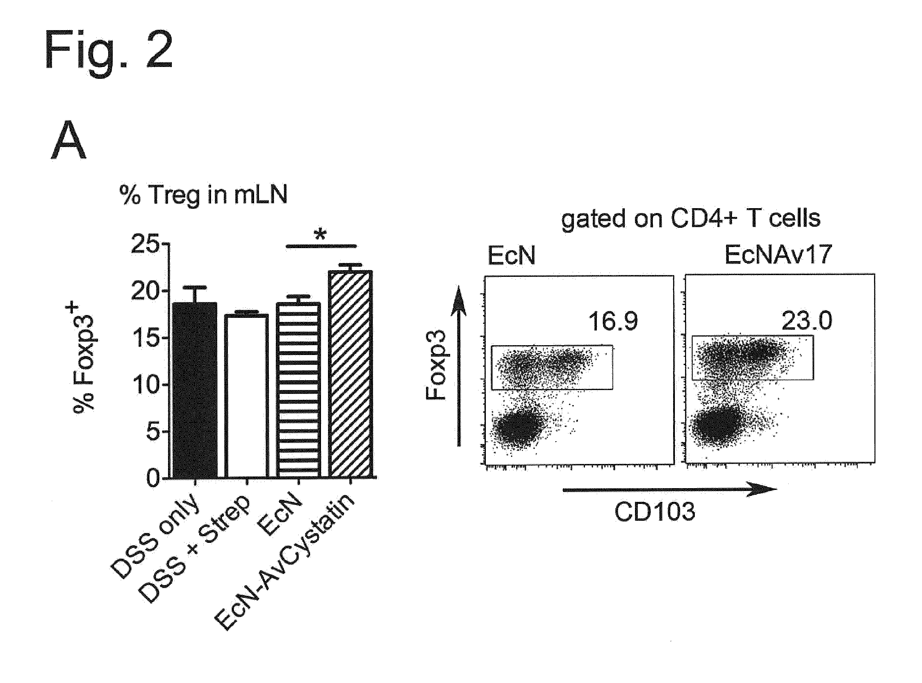

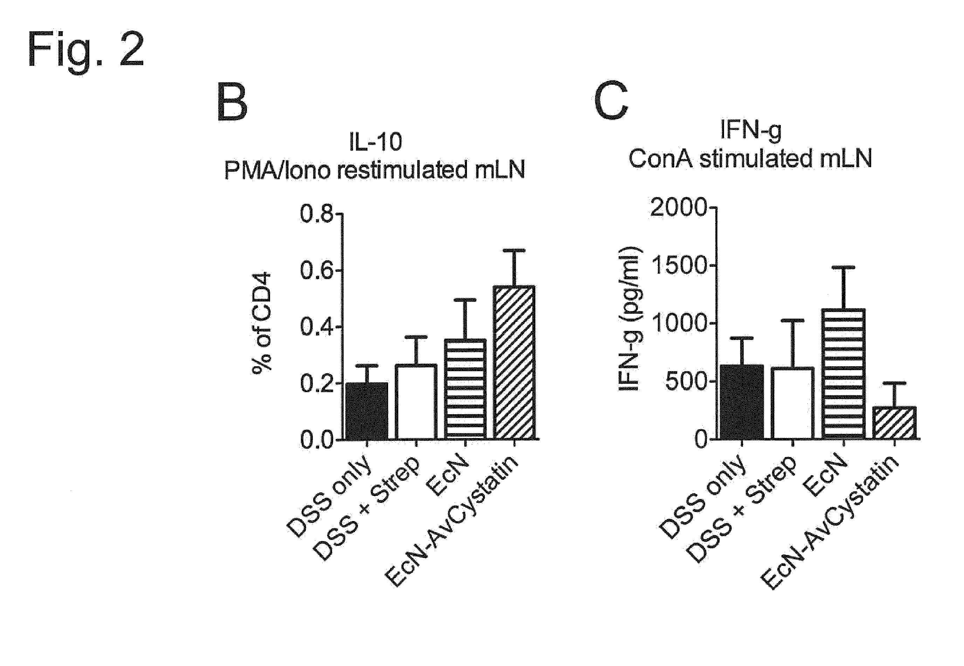

- **Figure 2:**: shows the effect of transgenic, parasite cystatin expressing, *E. coli* Nissle on a murine model of chronic colitis. A significant increase in% of FoxP3+ T cells was observed in the EcN-Cystatin treated group compared to the group receiving control EcN **(A).** In concanavalin A re-stimulated cells from the gut draining mesenteric lymph nodes (mLN) an increase in regulatory cytokine IL-10 **(B)** and a decrease in pro-inflammatory cytokine IFN-gamma was observed in the dextran sodium sulphate (DSS) induced colitic mice treated with EcN-Cystatin compared to the control groups receiving DSS alone (DSS) or treated with control *E. coli* Nissle (EcN) **(C).**

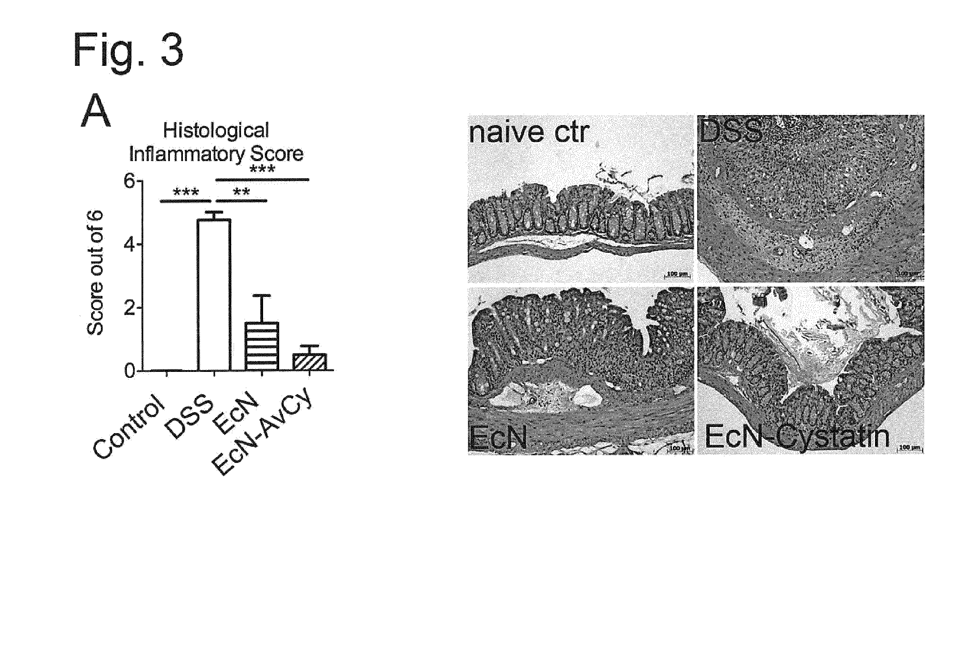

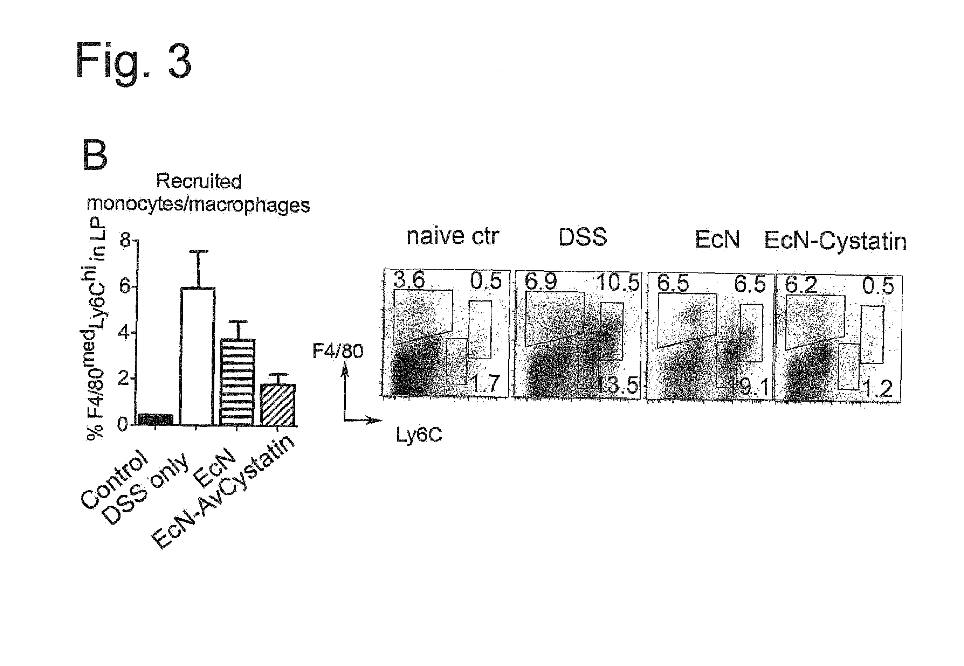

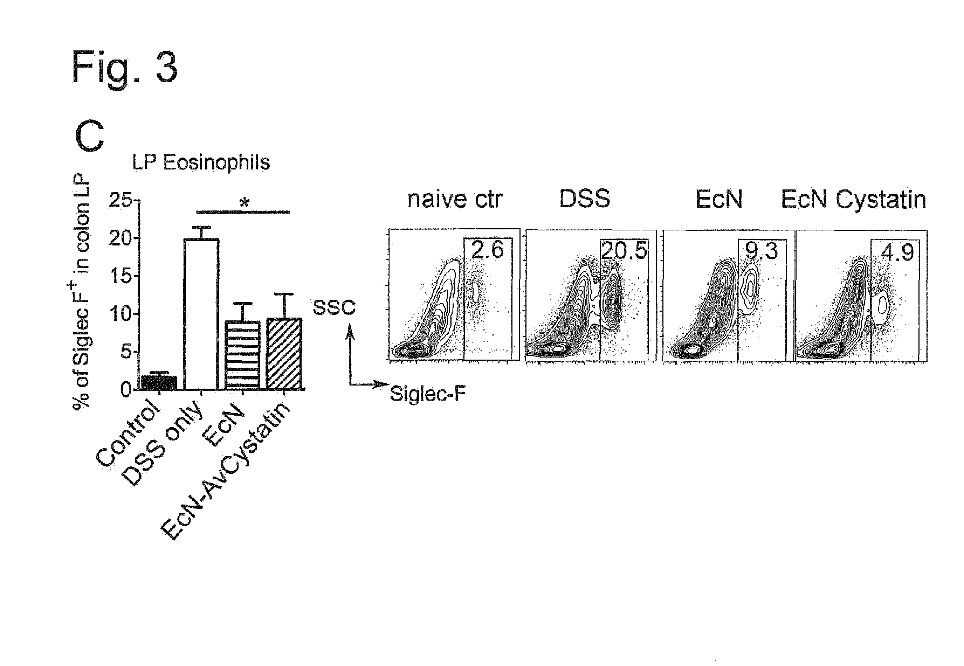

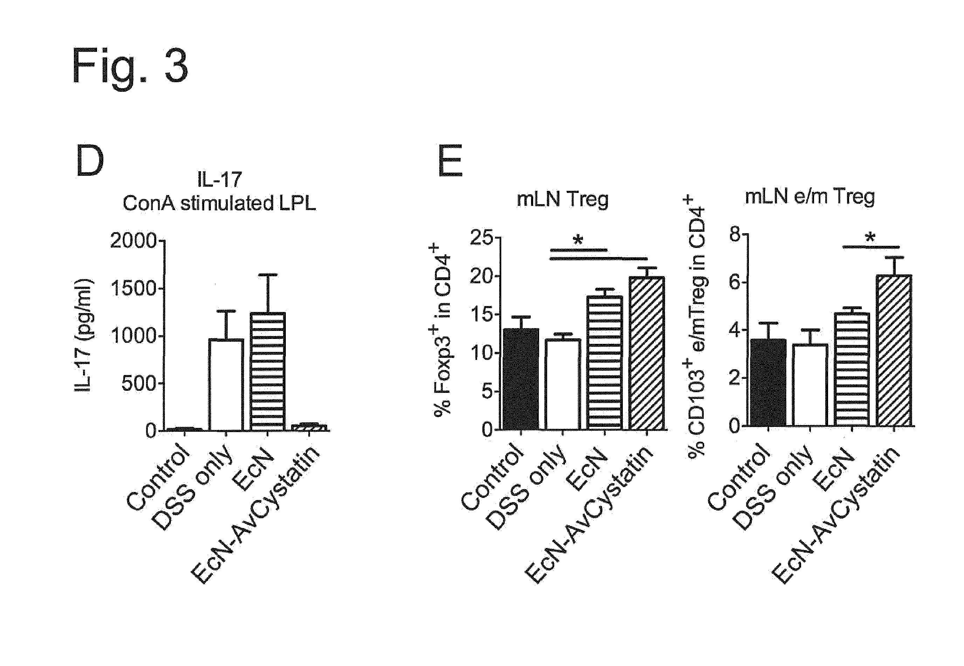

- **Figure 3:**: shows the effect of transgenic parasite cystatin expressing *E. coli* Nissle on a murine model of acute colitis. A significant decrease in inflammatory score of colon samples from DSS induced colitic mice treated with EcN-Cystatin was observed compared to the control group receiving DSS alone **(A).** A decrease in newly recruited monocytes/macrophages as determined by flow cytometry with colon lamina propria cells was also observed between the EcN-Cystatin treated group and DSS controls **(B).** Likewise infiltration of eospinophils in the lamina propria was reduced in the EcN-Cystatin treated group compared to DSS controls **(C).** Production of the pro-inflammatory cytokine IL-17 in Concanavalin A stimulated lamina propria lymphocytes was significantly lower in EcN-AvCystatin group compared with EcN treated controls **(D).** Percentage of Foxp3+ Tregs and CD103+Foxp3+ Tregs and effector memory (e/m) Tregs in the mesenteric lymph nodes was significantly higher in the EcN-AvCystatin treated group than in the DSS controls or the EcN treated animals **(E).**

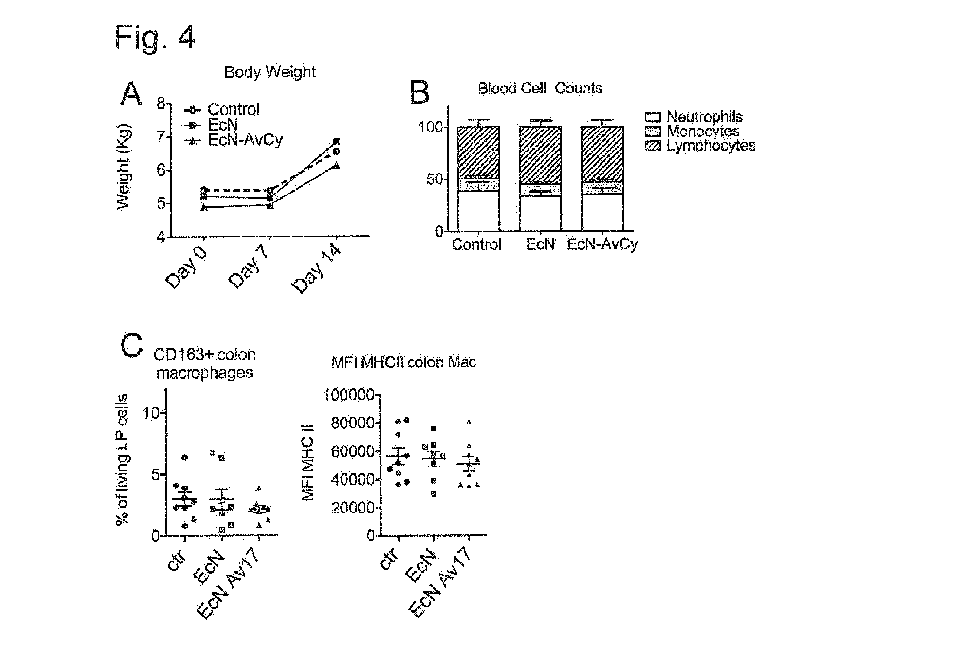

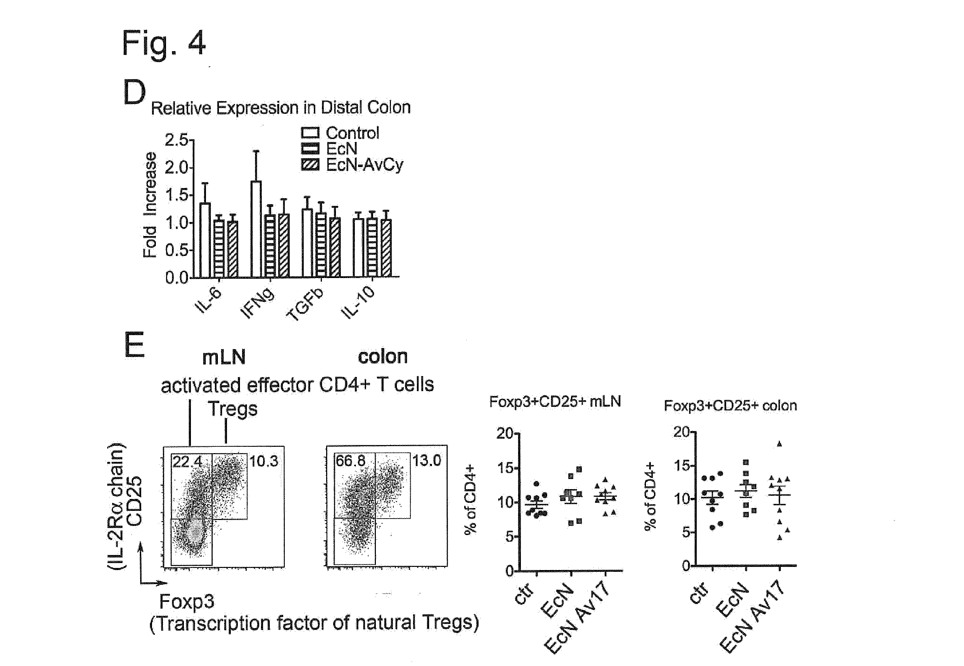

- **Figure 4:**: shows safety of transgenic parasite cystatin expressing *E*. *coli* Nissle in healthy young piglets. No significiant difference in body weight gain was observed for piglets fed EcN-AvCystatin, EcN or control piglets fed saline alone **(A).** Between treatment groups there was no significant difference between proportion of cell types counted in blood smears **(B)** or infiltration or activation of monocytes and macrophages as determined by flow cytometric analysis of CD163 and MHCII markers, respectively **(C).** There were no differences in expression of pro-inflammatory cytokines IL-6 and IFN-gamma or regulatory cytokines TGF-beta and IL-10 in the colon as determined with RT-PCR (D). There were no significant differences in percentage of Tregs in the mLN or colon between groups **(E).**

### EXAMPLES:

### Materials and methods:

### Construction of the Transgenic Probiotic

A spontaneously streptomycin resistant strain of EcN was gifted to us graciously by Dr. Tobias Ölschläger from the University of Würzburg. The strain also carries a kanamycin resistance cassette on the EcN specific plasmid pMUT2 and has a genetically modified version of the native EcN specific plasmid pMUT1. The modified pMUT1 plasmid, known as pMU13 was designed for functionality as a cloning vector through the addition of a tetracycline resistance cassette, a multiple cloning site and a hemolysin A secretion system. All Nsil cut sites outside of the hlyA gene were deleted to allow for insertion of genes within the hlyA component of the hemolysin A secretion system. This ensures that proteins expressed from the inserted gene will be secreted from the bacteria.

For insertion of AvCystatin into the pMUT13 vector primers were designed to add restriction enzyme, Nsil cut sites to both the 3' and 5' ends of the AvCystatin sequence when amplified (Fw 5'-ACGTATGCATTGGTGCGCTGTGAAGA-3' [SEQ ID NO. 5], Rv 5'-ACGTATGCATTCACTGATGAGAGTACT-3' [SEQ ID NO. 6]). The amplified AvCystatin gene was then inserted into the pMU13 vector at the Nsil cut site and transformed into chemically competent EcN. Successful clones were confirmed with AvCystatin specific primers (Fw 5'-TCGTGTCGACGGTTTTGGTGCGCTGTGAAGAAC-3' [SEQ ID NO. 7], Rv 5'-ACATGCGGCCGCTCACACTGATGAGAGTA-3' [SEQ ID NO. 8]) and western blot analysis of supernatants with AvCystatin specific antibodies.

### Animal Experimentation Protocol

Male, 9-11 week old C57BL/6 mice were assigned to one of four groups, a negative control group (Neg), a positive control group (Pos) an *E. coli* Nissle treated group (EcN) or a transgenic AvCystatin expressing *E. coli* Nissle treated group (EcN-AvCy). Animals in the Pos, EcN and EcN-AvCy group were treated for 7-9 days with 2.5-3.0% DSS in the drinking water. Immediately after the introduction to DSS and every 48hrs thereafter, EcN and EcN-AvCy groups were treated via oral gavage with 100/µl of 2x10¹⁰ CFU/ml *E. coli* Nissle or transgenic cystatin expressing *E. coli* Nissle in 0.9% saline, respectively. Animals in Neg and Pos groups were given 100µl of 0.9% saline via oral gavage at the same time points. Animals were euthanized before body weight loss reached 80%. All experiments were approved by the German ethics committee for the protection of animals.

### EcNlEcN-AvCy verification

Feces from all animals were collected one day before DSS and probiotic administration as well as at dissection. The previously described *E. coli* specific multiplex PCR (Blum-Oehler et al, 2003) was used to determine presence or absence of EcN, while an AvCystatin specific primer set (Fw 5'-TCGTGTCGACGGTTTTGGTGCGCTGTGAAGAAC-3' [SEQ ID NO. 9], Rv 5'-ACATGCGGCCGCTCACACTGATGAGAGTA-3' [SEQ ID NO. 10]) was used to amplify the transgene.

### Histology

Distal colon tissues were fixed in 3.7% formalin. The fixed samples were dehydrated, embedded in paraffin, mounted, stained with H&E and then scored for inflammation/tissue damage by Dr. A. Kühl, RCIS Charité, Berlin (Inflammation: (0) no inflammation; (1) increased number of inflammatory cells in lamina propria; (2) inflammatory cells extending into the submucosa; (3) transmural inflammatory infiltrates. Tissue damage: (0) no mucosal damage; (1) discrete epithelial lesion; (2) erosion or focal ulceration; (3) severe mucosal damage with extended ulcerations extending into the bowel wall.

### In Vitro Cell Culture

Spleen and mesenteric lymph nodes (mLN) were aseptically passed through cell strainers and washed in RPMI 1640 media containing 5% FCS (Biochrom, Germany), 20mM L-glutamine (PAA), 100U/mL penicillin and 100mg/mL streptomycin (PAA). Erythrocytes were removed with lysis buffer (0.01 M KHCO₃, 0.155M NH₄Cl, 0.1 mM EDTA, pH 7.5) for 5 min on ice, washed and taken up in RPMI 1640 containing 10% FCS. Cells were counted with a CASY counter (Innovatis, Germany) and 3.5x10⁵ cells /well were plated onto 96 well plates. Cells were stimulated with concanavalin A (2 µg/mL) in a final volume of 200µl. After 48hrs incubation at 37°C with 5% CO₂ supernatants were removed and stored in -20°C until further analysis.

### Cytokine Analysis

Murine specific ELISA antibody pairs for IL-10 (BD Biosciences), IL-17 (R&D Systems) and IFN-γ (eBioscience) were used according to manufacturer recommendations to determine the cytokine levels from the in vitro culture supernatants.

### Flow Cytometry

Fluorescence labeled anti-murine antibodies for markers Ly-6G (clone RB6-8C5), F4/80 (clone BM8), Ly-6C (clone HK1.4), MHCII (clone M5/114.15.2), Ki-67 (clone SolA15), CD4 (clone RM4-5), CD69 (clone H1.2F3), CD25 (clone PC61.5), CD103 (clone 2E7), and FoxP3 (FJK-16S) as well as fixable viability dye and rat IgG isotype controls (eBRG1) were obtained from eBioscience. Anti-murine, fluorescently labeled antibodies for Siglec-F (clone E50-2440) and CD11b (clone M1/70) were obtained from BD Bioscience. Anti FCγRII (clone 24G2) was gratefully received from the Deutsches Rheuma Forschungszentrum Berlin. Surface staining was performed in PBS, 0.2% BSA. For detection of Foxp3 and Ki-67 expression, cells were treated with the fixation/permeabilization buffers from eBioscience according to the manufacturer's instructions. Cells were acquired using an LSRFortessa cell analyzer (BD Biosciences) and analyzed with FloJo software (Treestar, USA).

### Statistics

The study includes 4 experiments with 3-5 animals per group. Groups were compared with one-way and two-way ANOVA as well as Mann-Whitney U test using GraphPad Prism software (San Diego, USA). Data is reported as means±SEM and differences were deemed significant if p<0.05.

### Results:

A native EcN-specific plasmid was modified to include a multiple cloning site for ease of gene insertion, an antibiotic resistance gene for selection purposes and a secretion system to allow inserted gene products to be secreted by the bacteria. The AvCystatin encoding gene was amplified using primers designed to add restriction enzyme cut sites to the 3' and 5' ends of the gene sequence. Using restriction enzyme digestion and ligation, the amplicon was then inserted in frame into the vector at a site that allows both expression and secretion of the functional protein upon transformation of the vector into EcN.

The cystatin-expressing EcN has been verified by both molecular, PCR based determination of the cystatin gene in extracted plasmid DNA (Fig. 1A), as well as with Western blot analysis of supernatant of bacterial culture (Fig. 1 B).

### The effect of a single application of nematode cystatin-expressing, transgenic EcN on a murine model of chronic colitis.

Adult male C57BL/6 mice were administered 3 cylces of 2.5% dextran sodium sulphate (DSS, a chemical disrupting the epithelial barrier of the intestine and thus leading to a colitic response) in the drinking water for 7 days with rest periods of 1 0 days between cycles. On day 3 8, the first day of the last DSS cycle, either 2x10⁹ CFU cystatin-expressing EcN (EcN-Cystatin), 2x10⁹ CFU control EcN, or saline was administered via oral administration. Colonization was supported through application of streptomycin in the drinking water on day 37, one day before bacterial administration until sacrifice on day 45. While histological inflammatory scores were not significantly improved (not shown) in the EcN-Cystatin groups compared to the EcN or saline control groups a significant increase in FoxP3+ regulatory T cells (Fig. 2A) and a trend towards increased production of regulatory cytokine IL-I 0 (Fig. 2B) was observed upon re-stimulation of lymphocytes from the gut draining mesenteric lymph nodes. Likewise, a trend towards decreased production of the inflammatory cytokine IFN-gamma was observed (Fig. 2C). This study, performed in a very harsh, challenging colitis model, shows the potential of EcN-Cystatin in ameliorating chronic colitis, as seen through the induction of regulatory cell types and cytokine expression and a suppression of inflammatory cytokine production.

### The effect of multiple applications of nematode cystatin-expressing, transgenic EcN on a murine model of acute colitis.

Adult male C57BL/6 mice were administered 1 cylce of 3% DSS in the drinking water for 8 days. Either 2xI09 CFU EcN-Cystatin, 2xI09 CFU EcN or saline was administered via oral administration every 2 days throughout the DSS application. Animals were sacrificed at day 8. There was a significant decrease in histological inflammatory score in animals treated with EcN or EcN-Cystatin compared with saline treated controls (Fig. 3A). The lowest inflammatory scores were observed in mice receiving EcN-Cystatin (Fig. 3A). A reduction in recruitment and activation of monocytes/macrophages, indicators of inflammation, to the colon lamina propria was observed in EcN-Cystatin treated groups compared to saline control animals (Fig. 3B). Likewise a decrease in eosinophilia (Fig. 3C) and inflammatory cytokine IL-17 (Fig. 3D) was also observed in the colon of EcN-Cystatin treated mice compared with the controls. Again, regulatory T cells in the mesenteric lymph nodes were significantly increased in the EcN-Cystatin treated group compared with EcN alone or saline treated controls (Fig. 3E). This trial demonstrates the potential of EcN-Cystatin in the attenuation of inflammation in acute colitis as demonstrated through the induction of regulatory cells and the down-regulation of inflammatory cell infiltration and pro-inflammatory cytokine expression.

### Safety of transgenic parasite Cystatin expressing E. coli Nissle in a porcine model

Healthy 24 day old male piglets were administered either IxI010 EcN-Cystatin, IxI010 EcN or saline control via oral syringe every 2nd day for 14 days. Animals were euthanized at day 14. No significant differences in weight gain (Fig. 4A), blood cell populations (Fig. 4B), recruitment and activation of colon macrophages (Fig. 4C), percentage of FoxP3⁺- regulatory T cells (Fig. 4D) or inflammatory and regulatory cytokine expression in the colon were observed between treatment groups (Fig. 4E). This study demonstrates the safety of EcN-Cystatin application in healthy monogastrics as a therapeutic for gastrointestinal inflammatory diseases.

## Claims

1. Transgenic probiotic microorganism genetically engineered to express and secrete nematode derived cystatin.

2. Transgenic probiotic microorganism of claim 1, wherein the probiotic microorganism is a probiotic bacterium.

3. Transgenic probiotic microorganism of claim 1, wherein the probiotic microorganism is selected from the bacterial genus lactobacillus, bifidobacterium, escherichia and/or from the yeast genus saccharomyces.

4. Transgenic probiotic microorganism of one of the preceding claims, wherein the probiotic microorganism is *Escherichia coli* strain Nissle 1917.

5. Transgenic probiotic microorganism of one of the preceding claims, wherein the nematode derived cystatin comprises or consists of:
- an amino acid sequence with SEQ ID NO. 1, 2 or 3; and/or
- an amino acid sequence that is at least 70% identical, preferably at least 80% identical, more preferably at least 90% identical and most preferably at least 99% identical to an amino acid sequence with SEQ ID NO. 1, 2 or 3.

6. Transgenic probiotic microorganism of one of the preceding claims, wherein the nematode derived cystatin comprises or consists of:
- an amino acid sequence with SEQ ID NO. 1; and/or
- an amino acid sequence that is at least 70% identical, preferably at least 80% identical, more preferably at least 90% identical and most preferably at least 99% identical to an amino acid sequence with SEQ ID NO. 1.

7. Pharmaceutical composition comprising a transgenic probiotic microorganism of one of claims 1 to 6 and at least one pharmaceutically acceptable excipient.

8. Transgenic probiotic microorganism of one of claims 1 to 6 or pharmaceutical composition of claim 7 for use in treatment of gastro-intestinal inflammatory diseases.

9. Transgenic probiotic microorganism of one of claims 1 to 6 or pharmaceutical composition of claim 7 for use in treatment of inflammatory bowel diseases.

10. Transgenic probiotic microorganism of one of claims 1 to 6 or pharmaceutical composition of claim 7 for use in treatment of Crohn's disease and/or ulcerative colitis.

11. Use of a transgenic probiotic microorganism of one of claims 1 to 6 or a pharmaceutical composition of claim 7 in the manufacture of a medicament for treatment of gastro-intestinal inflammatory diseases.

12. Use of claim 11, wherein the gastro-intestinal inflammatory disease is an inflammatory bowel disease.

13. Use of claim 11 or 12, wherein the gastro-intestinal inflammatory disease is Crohn's disease and/or ulcerative colitis.

14. Method of treatment of gastro-inflammatory diseases, wherein a subject in need of such treatment is administered an effective amount of a transgenic probiotic microorganism of one of claims 1 to 6 or of a pharmaceutical composition of claim 7.

15. Method of claim 14, wherein the gastro-inflammatory diseases to be treated are inflammatory bowel disease, preferably Crohn's disease and/or ulcerative colitis.
